# EUROPEAN PATENT APPLICATION

(11) **EP 2 213 318 A1**
(43) Date of publication of application: **04.08.2010**
(21) Application number: 08843356.0
(22) Date of filing: 31.10.2008
(51) Int. Cl.: A61M 5/00, A61B 8/12, A61M 25/00

(54) **DRUG SOLUTION-ADMINISTRATION SYSTEM AND CANNULA FOR ADMINISTERING DRUG SOLUTION**

(30) Priority: 31.10.2007 JP 2007284468; 01.05.2008 JP 2008119950
(71) Applicant: Olympus Corporation, Shibuya-ku Tokyo 151-0072 (JP)
(72) Inventor: TOGAWA, Tsuyoshi, Tokyo 151-0072 (JP)
(74) Representative: Gunzelmann, Rainer
(86) International application number: PCT/JP2008/069935
(87) International publication number: WO 2009/057774

(57) **Abstract**

A medicinal-solution administration system feeds a medicinal solution to a mammal including a human and includes a medicinal-solution feed device (1) that feeds a medicinal solution, an observation device (9) that includes an elongated observation probe (7) and observes a diseased part to which the medicinal solution is administered, and a medicinal-solution administration cannula (C) that has at least a part thereof implanted in a body, and the medicinal-solution administration cannula (C) guides the medicinal solution, which is fed from the medicinal-solution feed device (1), and the observation probe (7) to the diseased part. Thus, it is possible to achieve a medicinal-solution feed system and a medicinal-solution feed cannula that can reduce the burden, such as irradiation, on a patient and easily perform checking of the effect of the anticancer-agent administration.

## Description

### TECHNICAL FIELD

The present invention relates to a medicinal-solution administration system and a medicinal-solution administration cannula that continuously administer a medicinal solution, such as an anticancer agent, directly to a diseased part for a relatively long period of time.

### BACKGROUND ART

Conventionally, scheduled administrations are performed to administer an anticancer agent using an intravenous drip, an osmotic pump (see non-Patent Document 1) or the like. An administration via a vein or the like, which takes about four hours a day, is repeatedly performed for about a week. Such a scheduled administration is repeatedly performed while taking a break period of about two weeks, for example. Taking the effect of the anticancer-agent administration in the previous scheduled administration into consideration, actions such as changing the amount of medicinal solution or type of medicinal solution for the current scheduled administration are taken.

Patent document 1: Japanese Laid-open Patent Publication No. 2006-167298
Patent Document 2: International Publication No. WO 1993/021940 Pamphlet
Non-Patent Document 1: Theewes F and Yum SI.Principles of the operation of genericosmotic pump for the delivery of semisolid or liquid drug formulations.Ann Biomed Eng 1976,4(4): 343-353
Non-Patent Document 2: HIF-1 activation regulation mechanism by hypoxic response
   http://www.biology.tohoku.ac.jp/lab-www/molbiol/hypoxia/main.html
Non-Patent Document 3: Current medical practice information
   http:/kk.kyodo.co.jp/iryo/news/0822supersonic.html
Non-Patent Document 4: Non-invasive biological diagnosis by using light
   http://www.mext.go.jp/b_menu/shingi/gijyutu/gijyutu3/toushi n/07091111/008.htm
Non-Patent Document 5:
   http://www.invitrogen.co.jp/qdot/qtracker.shtml

### DISCLOSURE OF INVENTION

### PROBLEM TO BE SOLVED BY THE INVENTION

In order to check, after each scheduled administration, the effect of the anticancer-agent administration to a diseased part, a CT device or the like is conventionally used. A patient is irradiated with X-rays each time upon each scheduled administration; therefore, there is a problem in that the irradiation amount to the patient is considerable.

In addition, because a CT device is large-sized so that places to be installed are limited to large hospitals and the number of CT devices is also limited, there is a problem in that a considerable time and work is required for checking the effect of the anticancer-agent administration.

The present invention has been made in view of the above, and an object of the present invention is to provide a medicinal-solution administration system and a medicinal-solution administration cannula, with which the burden on a patient can be reduced and the effect of the administration of medical solutions including an anticancer-agent can be easily checked.

### MEANS FOR SOLVING PROBLEM

To solve the problem described above and achieve the object, a medicinal-solution administration system according to the present invention for feeding a medicinal solution to a mammal including a human includes a medicinal-solution feed device that feeds a medicinal solution; an observation device that includes an elongated observation probe and observes a diseased part to which the medicinal solution is administered; and a medicinal-solution administration cannula that has at least a part thereof implanted in a body, wherein the medicinal-solution administration cannula guides the medicinal solution, which is fed from the medicinal-solution feed device, and the observation probe to the diseased part.

In the medicinal-solution administration system according to the present invention as set forth in the invention described above, the medicinal-solution administration cannula has a common channel that is used as a channel for administering the medicinal solution and as a channel for inserting the observation probe, and an administration of the medicinal solution and an observation by the observation probe are performed at different timings.

In the medicinal-solution administration system according to the present invention as set forth in the invention described above, the medicinal-solution administration cannula separately has a channel for administering the medicinal solution and a channel for inserting the observation probe.

In the medicinal-solution administration system according to the present invention as set forth in the invention described above, the channel for administering the medicinal solution and the channel for inserting the observation probe are concentrically arranged.

In the medicinal-solution administration system according to the present invention as set forth in the invention described above, the channel for administering the medicinal solution and the channel for inserting the observation probe are arranged in parallel.

In the medicinal-solution administration system according to the present invention as set forth in the invention described above, the medicinal-solution administration cannula has a side opening and the medicinal solution is administered to the diseased part through the side opening.

In the medicinal-solution administration system according to the present invention as set forth in the invention described above, the medicinal-solution administration cannula has a marker that is to be detected by the observation probe and is arranged near the side opening.

In the medicinal-solution administration system according to the present invention as set forth in the invention described above, the observation probe is an ultrasonic probe, and the marker reflects ultrasound generated by the ultrasonic probe.

In the medicinal-solution administration system according to the present invention as set forth in the invention described above, the marker is an air bubble.

In the medicinal-solution administration system according to the present invention as set forth in the invention described above, the medicinal-solution administration cannula is rotatable around an axis of the medicinal-solution administration cannula.

In the medicinal-solution administration system according to the present invention as set forth in the invention described above, the side opening has a plurality of side openings arranged in a circumferential direction.

In the medicinal-solution administration system according to the present invention as set forth in the invention described above, the observation probe is rotatable around an axis of the observation probe while being accommodated in a channel into which the observation probe is inserted in the medicinal-solution administration cannula.

In the medicinal-solution administration system according to the present invention as set forth in the invention described above, the observation probe is an ultrasonic probe, and the medicinal-solution administration system includes a balloon arranged on an end of the probe, and an inserting unit that inserts fluid into the balloon.

In the medicinal-solution administration system according to the present invention as set forth in the invention described above, the observation probe is an imaging probe.

In the medicinal-solution administration system according to the present invention as set forth in the invention described above, the imaging probe concurrently uses a contrast agent.

In the medicinal-solution administration system according to the present invention as set forth in the invention described above, the imaging probe is a fluorescent probe.

In the medicinal-solution administration system according to the present invention as set forth in the invention described above, the imaging probe is a luminescence probe.

In the medicinal-solution administration system according to the present invention as set forth in the invention described above, the imaging probe is an ultrasonic probe.

In the medicinal-solution administration system according to the present invention as set forth in the invention described above, the observation probe is a bio-sensor probe.

In the medicinal-solution administration system according to the present invention as set forth in the invention described above, the observation probe includes a marker, and the medicinal-solution administration cannula includes a detecting unit that detects the marker and is provided in a channel into which the observation probe is inserted.

In the medicinal-solution administration system according to the present invention as set forth in the invention described above, the detecting unit detects a position of the observation probe in a relative radial direction and a relative thrust direction with respect to an axis of the medicinal-solution administration cannula.

In the medicinal-solution administration system according to the present invention as set forth in the invention described above, the marker is a magnetic marker.

In the medicinal-solution administration system according to the present invention as set forth in the invention described above, an elongated puncture needle that is inserted into the medicinal-solution administration cannula to administer the medicinal solution is further included, the puncture needle includes a marker, and the medicinal-solution administration cannula includes a detecting unit that detects the marker and is provided in a channel into which the puncture needle is inserted.

In the medicinal-solution administration system according to the present invention as set forth in the invention described above, the detecting unit detects a position of the puncture needle in a relative radial direction and a relative thrust direction with respect to an axis of the medicinal-solution administration cannula.

In the medicinal-solution administration system according to the present invention as set forth in the invention described above, the marker is a magnetic marker.

In the medicinal-solution administration system according to the present invention as set forth in the invention described above, the puncture needle has a curved area that can be curved near its end.

In the medicinal-solution administration system according to the present invention as set forth in the invention described above, the puncture needle is a multi-layer pipe formed with at least two or more pipes, an inner pipe is slidably inserted into an outer pipe in an axial direction of the outer pipe, the inner pipe can be protruded from an end of the outer pipe, a curved area is formed on an end of at least one pipe, the inner pipe has a smaller bending rigidity than the outer pipe, and the curved area is formed to be curved to fall within a limit of elasticity such that the inner pipe is elastically deformable inside the outer pipe.

In the medicinal-solution administration system according to the present invention as set forth in the invention described above, the observation device displays and outputs a position of the puncture needle in a relative radial direction and a relative thrust direction with respect to an axis of the medicinal-solution administration cannula, which is detected by the detecting unit.

In the medicinal-solution administration system according to the present invention as set forth in the invention described above, display output of the observation device is such that a position of the medicinal-solution administration cannula and a relative position of the puncture needle with respect to the medicinal-solution administration cannula are displayed in a superimposed manner on an observation result of the diseased part.

In the medicinal-solution administration system according to the present invention as set forth in the invention described above, the medicinal-solution feed device includes a medicinal-solution reservoir that accommodates at least medicinal solution and feeds the medicinal solution to a medicinal-solution administration side; a variable flow-rate pump that variably discharges to the medicinal-solution reservoir a drive liquid for pushing out the medicinal solution in the medicinal-solution reservoir; a drive-liquid reservoir that accommodates the drive liquid; and a control unit that controls a discharge flow rate of the variable flow-rate pump such that a medicinal-solution administration amount per unit of time becomes a predetermined administration amount.

A medicinal-solution administration cannula according to the present invention is configured to be implanted in a body, configured to guide a medicinal solution fed from a medicinal-solution feed device to a diseased part in order to administer the medicinal solution to the diseased part, and configured to guide an observation probe of the observation device, which observes the diseased part to which the medicinal solution is administered, to proximity of the diseased part in order to observe the diseased part, wherein the medicinal-solution administration cannula includes a side opening through which the medicinal solution is administered to the diseased part and a marker that is to be detected by the observation probe and is arranged near the side opening.

In the medicinal-solution administration cannula according to the present invention as set forth in the invention described above, the observation probe is an ultrasonic probe, and the marker reflects ultrasound generated by the ultrasonic probe.

In the medicinal-solution administration cannula according to the present invention as set forth in the invention described above, the marker is an air bubble.

In the medicinal-solution administration cannula according to the present invention as set forth in the invention described above, the medicinal-solution administration cannula is rotatable around an axis of the medicinal-solution administration cannula.

In the medicinal-solution administration cannula according to the present invention as set forth in the invention described above, the side opening has a plurality of side openings arranged in a circumferential direction.

In the medicinal-solution administration cannula according to the present invention as set forth in the invention described above, a common channel is used as a channel for administering the medicinal solution and as a channel for inserting the observation probe.

In the medicinal-solution administration cannula according to the present invention as set forth in the invention described above, a channel for administering the medicinal solution and a channel for inserting the observation probe are separately provided.

In the medicinal-solution administration cannula according to the present invention as set forth in the invention described above, the channel for administering the medicinal solution and the channel for inserting the observation probe are concentrically arranged.

In the medicinal-solution administration cannula according to the present invention as set forth in the invention described above, the channel for administering the medicinal solution and the channel for inserting the observation probe are arranged in parallel.

In the medicinal-solution administration cannula according to the present invention as set forth in the invention described above, an end cannula arranged on a side of the diseased part, a connection cannula arranged on a side of a medicinal-solution feed port of the medicinal-solution feed device, and a connection section that connects the end cannula to the connection cannula are further included, and the observation probe is inserted into the end cannula with the connection section disconnected.

In the medicinal-solution administration cannula according to the present invention as set forth in the invention described above, an end cannula arranged on a side of the diseased part, a connection cannula arranged on a side of a medicinal-solution feed port of the medicinal-solution feed device, and a connection section that connects the end cannula to the connection cannula are further included, and the observation probe is inserted into the end cannula with the connection section disconnected.

In the medicinal-solution administration cannula according to the present invention as set forth in the invention described above, the end cannula includes a cylindrical tube that has an end opened, and an inner-plug member that closes the end of the cylindrical tube.

In the medicinal-solution administration cannula according to the present invention as set forth in the invention described above, the end cannula further includes an outer-casing cannula that is cylindrical and guides the end cannula.

### EFFECT OF THE INVENTION

According to the present invention, a medicinal solution fed from a medicinal-solution feed device is guided to a diseased part, thereby allowing the medicinal solution to be administered to the diseased part, and an observation probe of an observation device is guided to the proximity of the diseased part, thereby allowing the observation of the diseased part. Thus, it is possible to reduce the burden, such as irradiation, on a patient and easily perform checking of the effect of the anticancer-agent administration.

### BRIEF DESCRIPTION OF DRAWINGS

FIG. 1 is a schematic diagram that illustrates the schematic configuration of a medicinal-solution administration system according to a first embodiment of the present invention.
FIG. 2 is a schematic diagram that illustrates the state where an observation probe is inserted into the medicinal-solution administration system illustrated in FIG. 1.
FIG. 3 is a block diagram that illustrates the detailed configuration of a medicinal-solution feed device.
FIG. 4 is a diagram that illustrates the state near a boundary area between a medicinal solution and a drive liquid in a medicinal-solution reservoir.
FIG. 5 is a schematic diagram that illustrates the schematic configuration of a modified example of the medicinal-solution administration system illustrated in FIG. 1.
   FIG. 6 is a cross-sectional view that illustrates an example of an end cannula illustrated in FIG. 5.
   FIG. 7 is a cross-sectional view that illustrates a different example of the end cannula illustrated in FIG. 5.
   FIG. 8 is a schematic diagram that illustrates the schematic configuration of a medicinal-solution administration system according to a second embodiment of the present invention.
   FIG. 9 is a transverse cross-sectional view that illustrates the detailed configuration near the end of an end cannula illustrated in FIG. 8.
   FIG. 10 is a cross-sectional view taken along line A-A of the end cannula illustrated in FIG. 9.
   FIG. 11 is an explanatory diagram that illustrates one example of the state where the position of the opening of the end cannula is changed depending on the state of cancer cells.
   FIG. 12 is a longitudinal cross-sectional view that illustrates the configuration near the end of the end cannula in which a reflection section illustrated in FIG. 8 is implemented by an air bubble.
   FIG. 13 is a diagram that illustrates the configuration in which a balloon is arranged on the end cannula illustrated in FIG. 8.
   FIG. 14 is a cross-sectional view that illustrates the configuration of an end cannula in which a plurality of openings is arranged.
   FIG. 15 is a cross-sectional view that illustrates the configuration of an end cannula in which a medicinal-solution channel and a probe channel are concentrically formed.
FIG. 16 is a longitudinal cross-sectional view that illustrates the configuration of an end cannula in which a medicinal-solution channel and a probe channel are formed in parallel.
FIG. 17 is a cross-sectional view taken along line B-B of the end cannula illustrated in FIG. 16.
FIG. 18 is a cross-sectional view that illustrates the state where an outer-casing cannula is arranged on the circumference of the end cannula illustrated in FIG. 8.
FIG. 19 is a diagram that illustrates an example of the formation of the end cannula illustrated in FIG. 8.

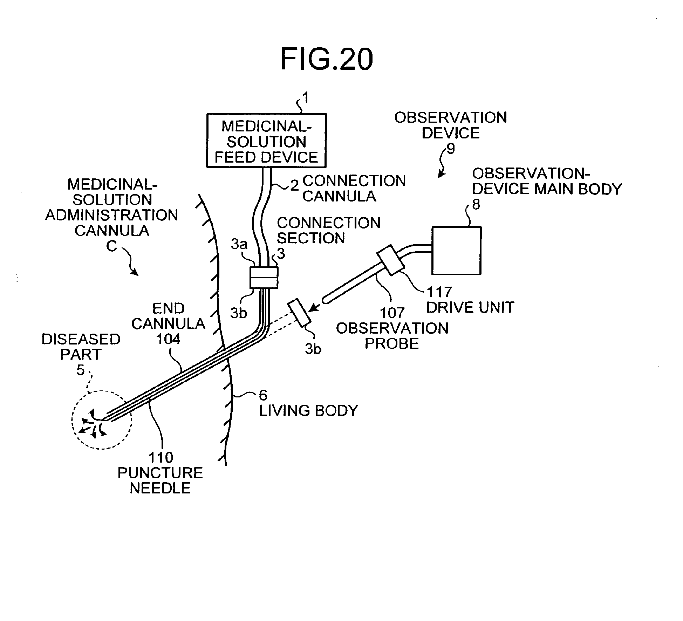

FIG. 20 is a schematic diagram that illustrates the schematic configuration of a medicinal-solution administration device according to an embodiment of the present invention.
FIG. 21 is a diagram that illustrates the configurations of the guide cannula, a puncture needle, and a detection probe illustrated in FIG. 20.
FIG. 22 is a cross-sectional view taken along line A-A illustrated in FIG. 21.
FIG. 23 is a cross-sectional view that illustrates the state where the puncture needle is inserted into the guide cannula.
FIG. 24 is a block diagram that illustrates the configuration of an observation-device main body unit.
FIG. 25 is a diagram that illustrates an example of a three-dimensional observation image.
FIG. 26 is a diagram that illustrates an example of the medicinal-solution administration that uses the detection probe and the puncture needle.

### EXPLANATIONS OF LETTERS OR NUMERALS

1 Medicinal-solution feed device
2 Connection cannula
3, 3a, 3b Connection section
4, 54, 54a, 74, 84, 104 End cannula
5 Diseased part
6 Living body
7, 107 Observation probe
8 Observation-device main body
9 Observation device
10 Medicinal-solution reservoir
11 Magnetic marker
12 Electro-osmotic-flow pump
13 Drive-liquid reservoir
20 Magnetic line sensor
21 Control unit
21a Biological-information detecting unit
21b Flow-rate change detecting unit
21c Pump control unit
22 Input/output unit
23 Storage unit
23a Biological-information/flow-rate relation storage unit
24 Biological-information sensor
30 Drive liquid
31 Medicinal solution
32a, 32b Oil
33a, 33b Air
44a, 45a Medicinal-solution channel
44b, 45b Probe channel
54b Silicon resin
57 Ultrasonic probe
57a, 107b Transmitting and receiving unit
61, 61a to 61c, 91 Opening
62, 62a to 62c, 63, 92 Reflection section
71 Balloon
72 Feed hole
100 Outer-casing cannula
107a, 110a, 130a Marker
110, 130 Puncture needle
111, 111a to 111d Encoder
112, 133 Opening
117 Drive unit
121 OR circuit
122 Counting unit
123 Thrust-position calculating unit
124 Radial-position calculating unit
125 Display processing unit
130 Puncture needle
131 Outer pipe
132 Inner pipe
C Medicinal-solution administration cannula
CA Cancer cell

### BEST MODE(S) FOR CARRYING OUT THE INVENTION

A detailed explanation will be given below of preferred embodiments of a medicinal-solution administration system and a medicinal-solution administration cannula according to the present invention with reference to the drawings. The present invention is not limited to these embodiments.

### First embodiment

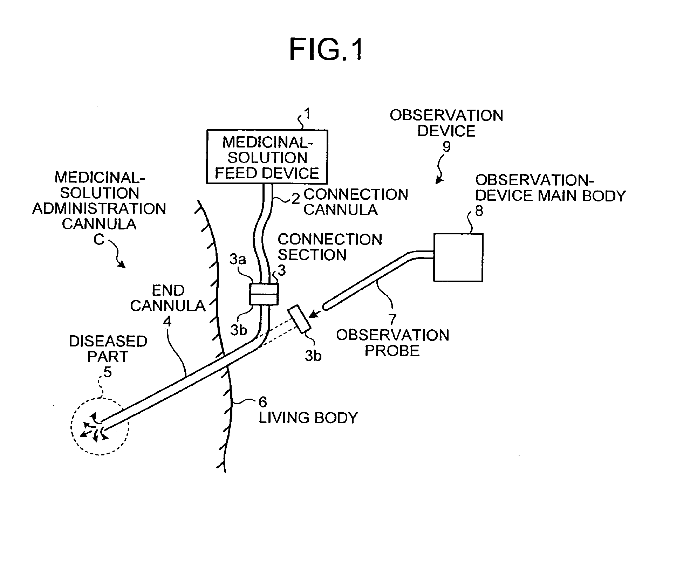

FIG. 1 is a schematic diagram that illustrates the schematic configuration of a medicinal-solution administration system according to a first embodiment of the present invention. The medicinal-solution administration system in FIG. 1 performs a scheduled administration by continuously and intensively discharging a medicinal solution including an anticancer agent, such as fluorouracil (5-FU), of about tens of ml to a diseased part 5 including a cancer in a living body 6, such as a human body for a long period of time of about one week. The system also enables an observation of the diseased part after the scheduled administration.

The medicinal-solution administration system includes a medicinal-solution feed device 1 that discharges a medicinal solution, an observation device 9 such as an ultrasonograph that includes an observation probe 7 such as an ultrasonic probe that observes the state of the diseased part 5, and a medicinal-solution administration cannula C that guides the medicinal solution discharged by the medicinal-solution feed device 1 to the diseased part 5 and guides the observation probe 7 to the diseased part 5. The medicinal-solution administration cannula C includes a connection cannula 2 arranged on the side of the medicinal-solution feed device 1, an end cannula 4 arranged on the side of the diseased part 5 and implanted in the living body, and a connection section 3 that connects the connection cannula 2 to the end cannula 4. An insertion port for the observation probe 7 is visible on the connection section 3 when the connection cannula 2 and the end cannula 4 are separated from each other. The observation device 9 includes the above-described observation probe 7 and an observation-device main body 8 that includes an undepicted display unit that displays observation information acquired by the observation probe 7, an undepicted processing unit that processes the observation information, and the like. The medicinal-solution feed device 1 may be implanted in the living body 6.

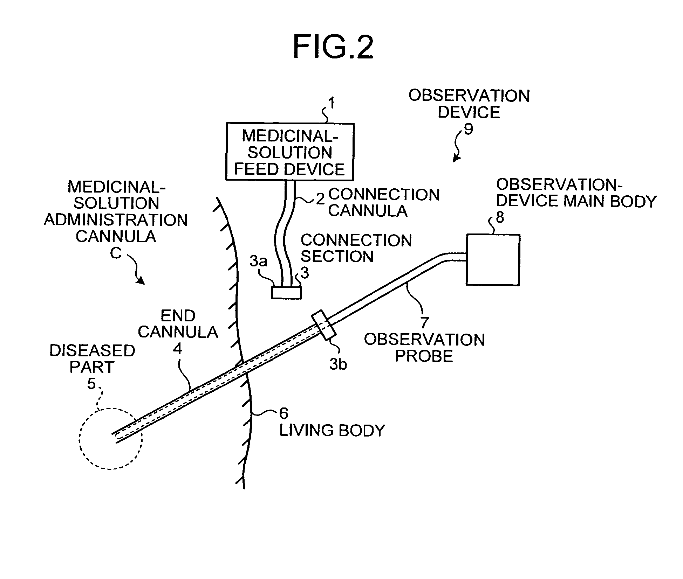

As illustrated in FIG. 1, the end cannula 4 is inserted into the living body 6 and, in the state where the tip section of the end cannula 4 has reached the inside of the diseased part 5, the medicinal solution is administered in a scheduled manner from the medicinal-solution feed device 1 to the diseased part 5 via the connection section 3 and the connection cannula 2. When the scheduled administration has finished, as illustrated in FIG. 2, the connection section 3 is separated and the observation probe 7 is inserted into the tip section of the end cannula 4 through the insertion port of a connection section 3b on the side of the end cannula 4 so that the state of the diseased part 5 is observed. For example, if the observation device 9 is an ultrasonograph and the observation probe 7 is an ultrasonic probe, ultrasonic cross-sectional images of the diseased part 5 can be obtained.

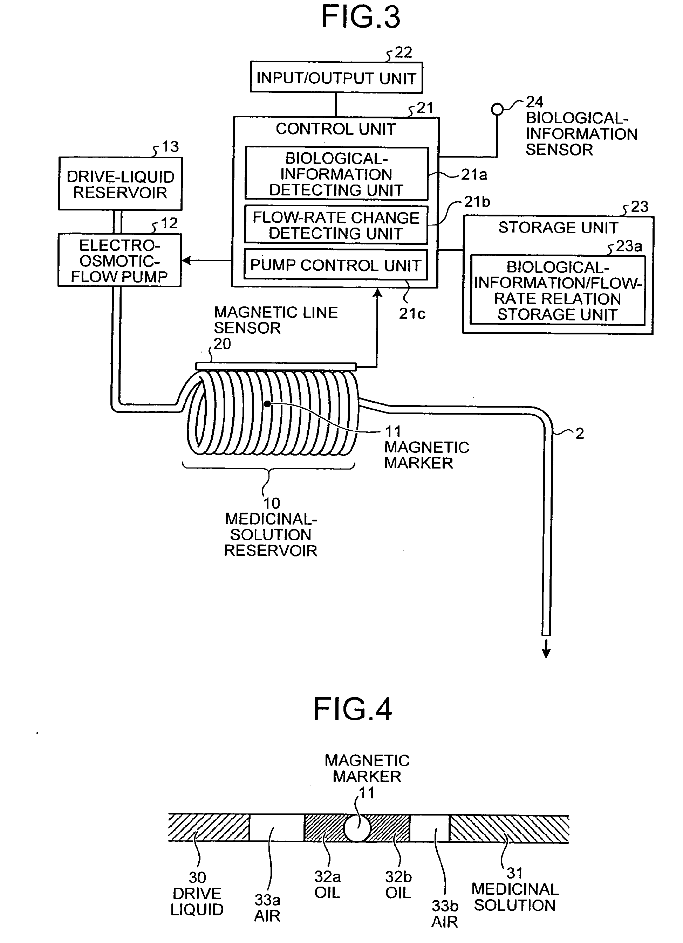

An explanation will be given of the detailed configuration of the medicinal-solution feed device 1 with reference to FIG. 3. As illustrated in FIG. 3, the medicinal-solution feed device 3 includes a medicinal-solution reservoir 10 in which a medicinal solution is accommodated, a drive-liquid reservoir 13 in which a drive liquid for pushing out the medicinal solution is accommodated, and an electro-osmotic-flow pump 12 that is arranged between the drive-liquid reservoir 13 and the medicinal-solution reservoir 10 and pushes out the drive liquid for pushing out the medicinal solution. It is preferable that ultrapure water or the like is used as the drive liquid. The operating principle of the electro-osmotic-flow pump 12 is such that the surfaces inside fine pores of an electro-osmotic material (porous material) are charged negatively, positive ions are in a large excess near the surfaces, and the positive ions are moved because they are subjected to forces due to an electric field applied from the outside so that the drive liquid flows, whereby the drive liquid is discharged. Therefore, the discharge amount of the drive liquid can be changed by changing the intensity of the electric field.

The medicinal-solution reservoir 10 is arranged such that a thin pipe with a uniform inner diameter is tightly wound like a coil with a uniform diameter, and a medicinal solution such as an anticancer agent is accommodated in the pipe. The drive liquid enters the pipe on the side of the electro-osmotic-flow pump 12. Specifically, the drive liquid is pushed out so that the medicinal solution is indirectly pushed out from the medicinal-solution reservoir 10.

In the medicinal-solution reservoir 10, a magnetic marker 11 is arranged on the end area of drive liquid 30. As illustrated in FIG. 4, the magnetic marker 11 is sandwiched between oil 32a and 32b, the outer sides of the oil 32a and 32b are sandwiched between air 33a and 33b, and the outer sides of the air 33a and 33b are filled with the drive liquid 30 and a medicinal solution 31. A magnetic line sensor 20 is arranged in an axial direction on the outer surface of the medicinal-solution reservoir 10 in an axial direction. As a result, the magnetic marker 11 is moved in accordance with the discharge of the medicinal solution, and the moving distance or the moving state of the moving magnetic marker 11 is detected by the magnetic line sensor 20.

The medicinal-solution feed device 1 includes a biological-information sensor 24. The biological-information sensor 24 detects biological information about the living body 6 as a medicinal-solution administration target. The biological information is, for example, in addition to a general index such as heart rate or blood pressure, a deviation enzyme such as γGTP, ALT, or AST that is increased during acute liver disorder, PaO2 that is a blood oxygen partial pressure, CRP that is an inflammation marker, or the like, and is information relating to the sensitivity of the medicinal solution to be administered, the effect on a target site, and the side-effect on a non-target site.

Moreover, the medicinal-solution feed device 1 includes a control unit 21 that performs at least the flow-rate control of the drive liquid by using the electro-osmotic-flow pump 12 in accordance with the detection result of the magnetic line sensor 20 and the detection result of the biological-information sensor 24, an input/output unit 22 that performs various input instructions or settings to the medicinal-solution feed device 1 and performs outputs such as display, and a storage unit 23 that includes a biological-information/flow-rate relation storage unit 23a that stores therein the relation between a biological-information value detected by the biological-information sensor 24 and the flow rate of the medicinal solution per unit time. The biological-information/flow-rate relation storage unit 23a may include a time profile that is the time change of the flow rate per unit time.

A biological-information detecting unit 21a of the control unit 21 detects a biological-information value on the basis of the value detected by the biological-information sensor 24. Furthermore, the magnetic line sensor 20 detects the position of the magnetic marker 11 so that a flow-rate change detecting unit 21b of the control unit 21 detects the flow-rate change of the medicinal solution. A pump control unit 21b of the control unit 21 obtains the flow rate per unit time on the basis of the biological-information value detected by the biological-information detecting unit 21 and the biological-information/flow-rate relation stored in the biological-information/flow-rate relation storage unit 23a and controls the flow rate of the drive liquid discharged by the electro-osmotic-flow pump 12, that is, controls the flow rate of the medicinal solution such that the flow rate change detected by the flow-rate change detecting unit 21b becomes the obtained flow rate per unit time.

Although the above-described control unit 21 controls the flow rate of the medicinal solution on the basis of the biological information acquired by the biological-information sensor 24, the flow rate control of the scheduled administration may be performed without the biological-information sensor 24.

In the first embodiment, because the medicinal-solution administration cannula C is used so that the end cannula 4 that is a pipe line for administering the medicinal solution to only the diseased part 5 is commonly used as the insertion path for the observation probe 7, it is possible to, with a simple configuration, cause the observation probe 7 to easily reach the diseased part 5 that is an observation target and observe the diseased part 5 directly and reliably. Particularly, because the end of the observation probe 7 reaches the inside of the diseased part 5, it is possible to observe the diseased part 5 from the inside of the diseased part 5 and observe the state of the diseased part 5 in detail.

Furthermore, compared to the diseased-part observation by using a CT device, a simplified observation can be performed, and the adverse effect on a living body due to the observation can be eliminated because there is no irradiation with X-rays. Therefore, the observation of the diseased part 5 by using the observation probe 7 can be conducted as needed and frequently.

Moreover, because the medicinal solution is administered to only the diseased part 5, it is unlikely to generate any side-effects due to the medicinal-solution administration. Furthermore, because the medicinal solution is administered from the inside of the diseased part 5, it is possible to perform the medicinal-solution administration on the entire diseased part 5 and perform the medicinal-solution administration on the diseased part 5 reliably and effectively.

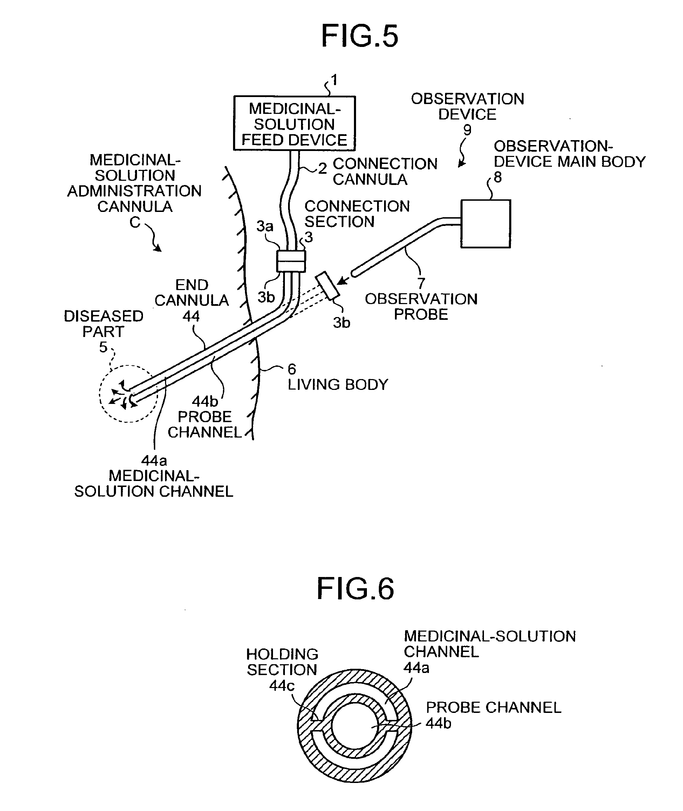

An explanation will be given of a modified example of the first embodiment with reference to FIG. 5. Although the medicinal-solution pipe line inside the end cannula 4 is used as the insertion path for the observation probe in the above-described first embodiment, in the modified example, a medicinal-solution channel 44a through which the medicinal solution passes and a probe channel 44b into which the observation probe 7 is inserted are arranged inside an end cannula 44 that corresponds to the end cannula 4.

Even if the medicinal-solution channel 44a and the probe channel 44b are separately arranged inside the end cannula 44, the end of the observation probe 7 can easily reach the diseased part 5 in the same manner as the first embodiment.

FIG. 6 illustrates an example of the specific configuration of the end cannula 44. Two pipes are formed that are concentric with each other. The space formed by the inner pipe is the probe channel 44b into which the observation probe 7 is inserted and the space formed between the inner pipe and the outer pipe is the medicinal-solution channel 44a through which the medicinal solution passes. A holding section 44c that holds the inner pipe is formed between the inner pipe and the outer pipe. Because of the holding section 44c, the end cannula 44 can be molded by extrusion, or the like, whereby the formation of the end cannula 44 is simplified.

In this case, the two medicinal-solution channels 44a are formed, and it is possible to perform multiagent treatment by feeding different medicinal solutions corresponding to the respective medicinal-solution channels 44a. In the multiagent treatment, a plurality of anticancer agents is administered in combination, and it is undesirable to mix the anticancer agents; however, because the plurality of medicinal-solution channels 44a is used, the anticancer agents can be administered while preventing the mixing of the anticancer agents. Conversely, if the medicinal-solution channels 44a are to be used as one medicinal-solution channel, at least parts of the holding section 44c on the side of the end of the end cannula 44 and on the side of the connection section 3b are cut out, or the like, to provide one medicinal-solution channel.

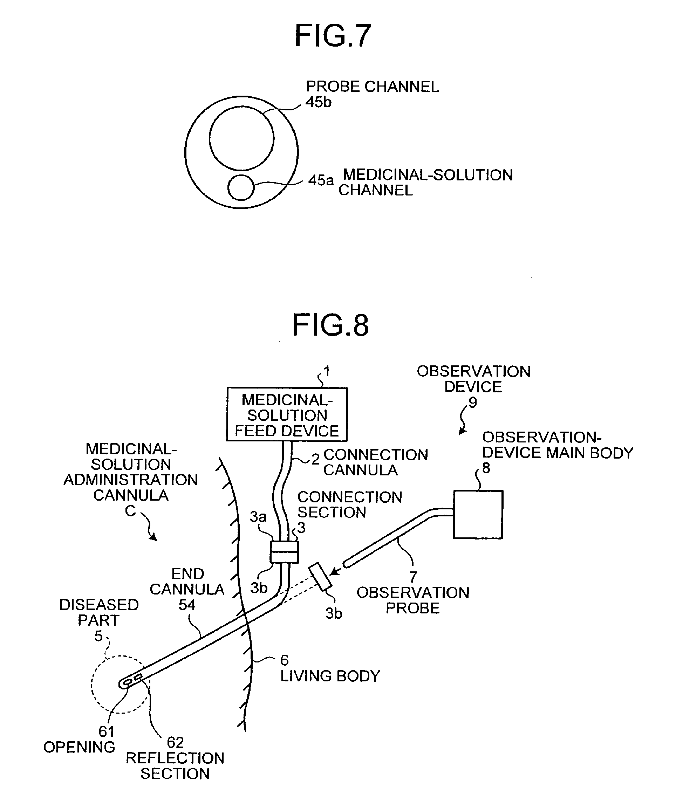

Furthermore, as illustrated in FIG. 7, a medicinal-solution channel 45a and a medicinal-solution channel 45b may be arranged adjacent to each other in parallel inside the end cannula 44.

### Second embodiment

Next, an explanation will be given of a second embodiment of the present invention. Although the ends of the end cannulas 4 and 44 have the openings in the above-described first embodiment, in the second embodiment, as illustrated in FIG. 8, the end of an end cannula 54 is closed, an opening 61 for administering the medicinal solution is arranged in a radial direction from the proximity of the end, and a reflection section 62 that reflects a physical quantity generated by the observation probe 7 is arranged at a position that corresponds to the position of the opening 61. The other configurations are the same as the first embodiment, and the same reference numerals are attached to the same components.

With such a configuration, the position of the medicinal-solution administration from the opening 61 can be selected relative to a circumferential direction. Furthermore, the positioning of the end of the observation probe to be inserted is facilitated. Moreover, because the reflection section 62 is arranged, the reflection section 62 is detected by the observation probe 7 so that the position of the opening 61 can be easily detected. It is preferable that the opening 61 and the reflection section 62 are arranged corresponding to each of the medicinal-solution channels 44a.

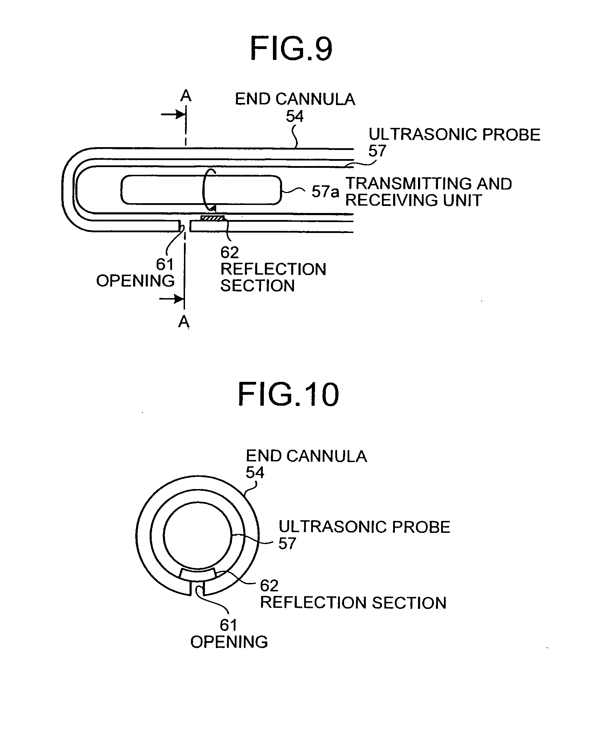

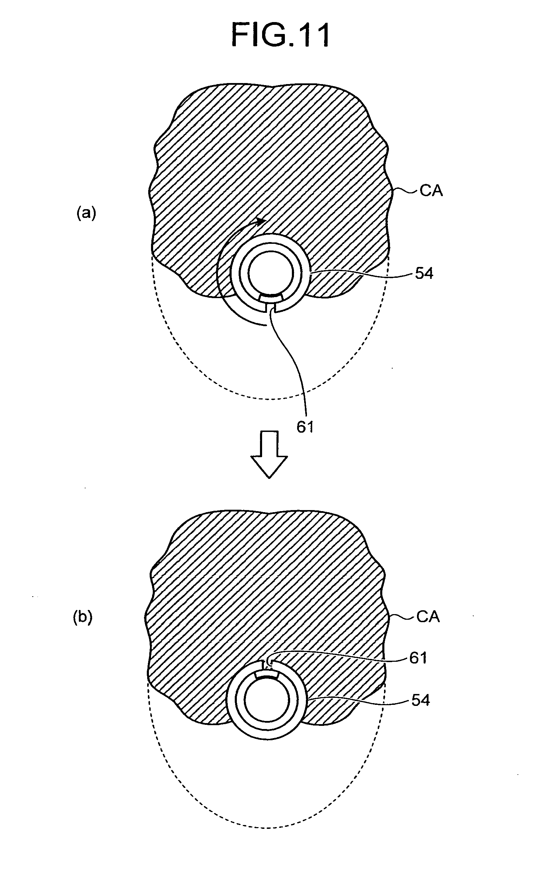

Specifically, as illustrated in FIGS. 9 and 10, the opening 61 is arranged in a circumferential direction near the end of the end cannula 54, and the medicinal solution is administered to the diseased part 5 from the opening 61. In the case where the observation probe 7 is an ultrasonic probe 57, the reflection section 62 implemented by a metal foil that reflects ultrasound is arranged near the opening 61. A transmitting and receiving unit 57a of the ultrasonic probe 57 is rotated and moved in an axial direction so that the reflection section 62 is detected, whereby it can be detected that the opening 61 exists at the position (in an axial direction and a circumferential direction) of the reflection section 62. The position of the opening 61 and the state of the observed diseased part 5 are compared, whereby the position of the medicinal-solution administration, i.e., the position of the opening 61, can be surely changed in accordance with the state of the diseased part 5. For example, as illustrated in FIG. 11(a), if cancer cells CA have disappeared near the opening 61 that is the position of the medicinal-solution administration and the cancer cells CA have grown near the back side of the position of the medicinal-solution administration, the end cannula 54 is rotated 180 degrees so that, as illustrated in FIG. 11(b), the opening 61 faces the area where the cancer cells CA have grown, whereby the medicinal solution can be delivered in an effective manner.

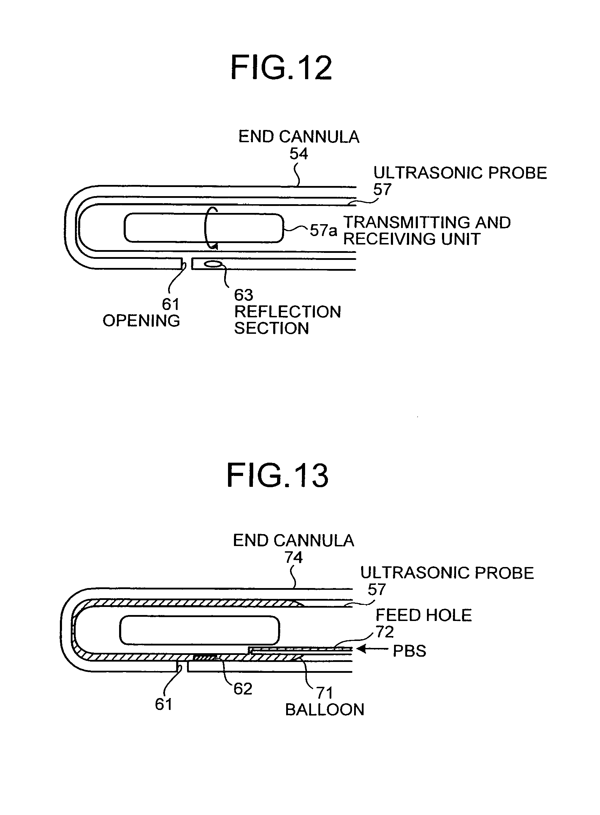

In the case where the ultrasonic probe 54 is used as the observation probe 7, because it is only necessary for the reflection section 62 to reflect ultrasound, as illustrated in FIG. 12, a reflection section 63 may be formed with an air bubble. With respect to the reflection section 63, it is only necessary to enclose air at the position of the reflection section 63 upon the formation of the end cannula 54. Because the reflection section 63 does not have protrusions inside or outside the end cannula 54, it is possible to smoothly perform the insertion of the end cannula into the living body 6 or the insertion of the ultrasonic probe 57.

Moreover, in the case of using the ultrasonic probe 57, if there is a space between the inside wall of an end cannula 74 and the surface of the end of the ultrasonic probe 57, the ultrasound is reflected due to the space and the observation of the diseased part 5 cannot be conducted; therefore, as illustrated in FIG. 13, it is preferable that a balloon 71 that covers the tip section of the ultrasonic probe 57 and a feed hole 72 that connects the connection section 3b to the inside of the balloon 71 are arranged and the balloon 71 is filled with PBS (Phosphate Buffered Saline) via the feed hole 72 during the observation so that the air space is not formed. The balloon 71 may be filled not only with the PBS but also, for example, pure water.

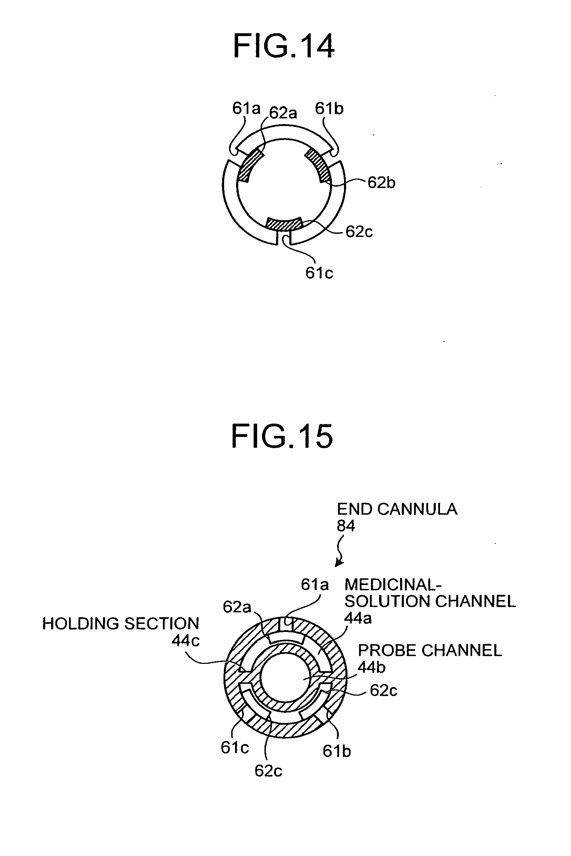

Although the one opening 61 and the one reflection section 62, 63 are arranged in the above-described second embodiment, as illustrated in FIG. 14, a plurality of openings 61a to 61c may be arranged on the circumference and a plurality of reflection sections 62a to 62c may be arranged at positions corresponding to the positions of the openings 61a to 61c. Moreover, the positions of the openings 61a to 61c and the reflection sections 62a to 62c may be located at positions that shift, respectively, in an axial direction. Each of the openings 61a to 61c may be identified by the position shift in the axial direction. Furthermore, each of the openings 61a to 61c may be identified by changing the sizes of the reflection sections 62a to 62c.

In the second embodiment, the medicinal-solution channel 44a and the probe channel 44b may be separately arranged. For example, as illustrated in FIG. 15, two pipes are formed that are concentric with each other via the holding section 44c so that the medicinal-solution channel 44a and the probe channel 44b are formed inside an end cannula 84. In FIG. 15, the plurality of openings 61a to 61c and reflection sections 62a to 62c is formed.

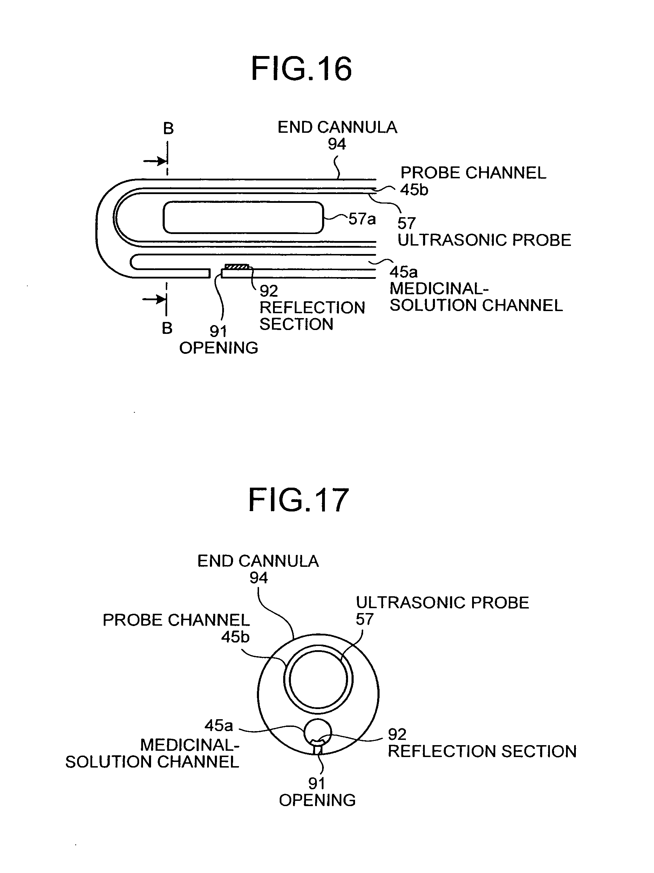

Similarly, as illustrated in FIGS. 16 and 17, the medicinal-solution channel 45a and the probe channel 45b may be arranged adjacent to each other in parallel inside an end cannula 94. In this case, one opening 91 and one reflection section 92 are arranged.

Although the surface of the end cannula is brought into contact with the living body in each of the above-described first and second embodiments, the present invention is not limited to this and an outer-casing cannula 100 that covers the outer surface of the end cannula may be further arranged. In this case, the outer-casing cannula 100 is first implanted in the living body 6 and the end cannula 54 is inserted into the outer-casing cannula 100.

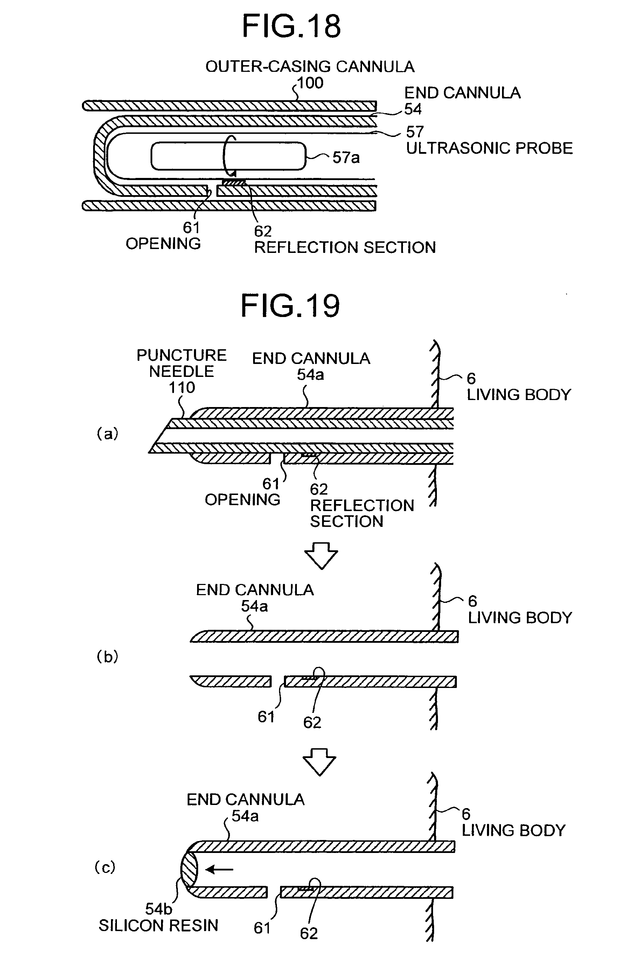

An explanation is given in the above-described first and second embodiments with the state where the end cannula 4 is implanted in the living body 6. In the case where the end cannula 4 is implanted in the living body 6, as illustrated in FIG. 19, the use of a puncture needle 110 facilitates the implant. In using the puncture needle 110, first, as illustrated in FIG. 19(a), an end cannula 54a and the puncture needle 110 are integrally inserted into the living body 6 from their end side. The end cannula 54a is cylindrical with its end formed to be tapered toward the center of the cylinder. The puncture needle 110 is inserted into the end cannula 54a, protruded to the end side from the end of the end cannula 54a, and has its end formed to be tapered. Afterwards, when the end reaches the diseased part 5, the puncture needle 110 is removed (see FIG. 19(b)). In this case, when the puncture needle 110 is removed, the observation probe 7 may be inserted to detect the reflection section 62 and the diseased part 5 so that the end position is confirmed. Then, as illustrated in FIG. 19(c), silicon resin 54b as an inner-plug member is inserted into the end opening from the side of the connection section 3b and the end area is closed so that the end cannula 54 illustrated in the second embodiment is formed. If the silicon resin 54b is not formed, the end cannula 4 illustrated in FIG. 19(b) is obtained, which corresponds to the first embodiment.

Although an explanation is given in the above-described first embodiment by using the ultrasonic probe 57 as an example of the observation probe 7, the present invention is not limited to this and the observation probe 7 may be anything as long as it can observe the diseased part 5. For example, OCT (optical coherence tomography) may be used. Furthermore, an endoscopic probe may be used. In the case where an endoscopic probe is used, for example, the end of the endoscopic probe is moved in an axial direction to detect the outer edge of the diseased part 5 so that the shape variation can be detected, and, in addition, the color alternation of the diseased part 5 is observed so that an angiogenesis state of the diseased part 5, which cannot be observed in ultrasonic cross-sectional images, can be grasped.

### Third embodiment

Next, an explanation will be given of a third embodiment of the present invention. A medicinal-solution administration apparatus according to the third embodiment includes the puncture needle 110 that is connected to the connection cannula 2 and administers the medicinal solution discharged by the medicinal-solution feed device 1 to the diseased part 5 and an end cannula 104 corresponding to the end cannula 4 that guides the puncture needle 110 to the diseased part 5, guides the observation probe 7 to the diseased part 5, and detects the relative position of the puncture needle 110 and the observation probe 7 with respect to the end cannula 4. The puncture needle 110 arranged on the end side of the connection cannula 2 connected to the side of the medicinal-solution feed device 1 and the observation probe 7 are alternately inserted into the end cannula 104 via the insertion port of the connection section 3b. Although the end cannula 4 has the medicinal-solution administration function and the observation-probe guide mechanism in the first embodiment, in the second embodiment, the puncture needle 110 has the medicinal-solution administration function and the end cannula 104 has the guide mechanism with respect to the puncture needle 110 and the observation probe 7.

Moreover, the observation device 9 includes an observation probe 107 corresponding to the observation probe 7 and a drive unit 117. The observation probe 107 is moved in a stepwise manner or at a constant speed in the axial direction of the end cannula 104 by the drive unit 117 while observation images are acquired. Furthermore, the observation-device main body 8 includes an undepicted display unit that displays observation information, such as ultrasonic cross-sectional images obtained by the observation probe 107, an undepicted processing unit that processes the observation information, and the like. The other configurations are the same as those of the first embodiment, and the same reference numerals are attached to the same components.

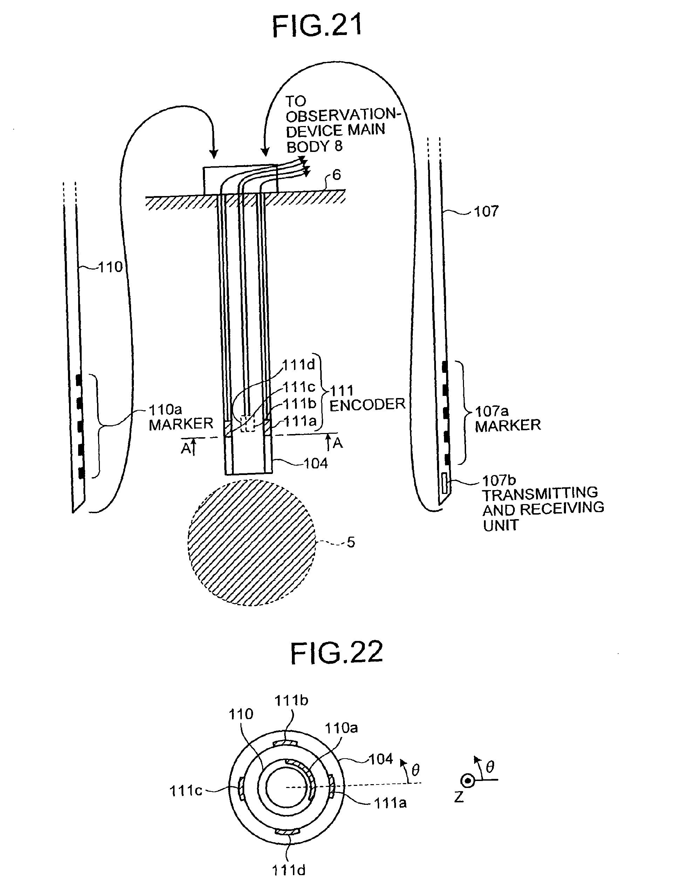

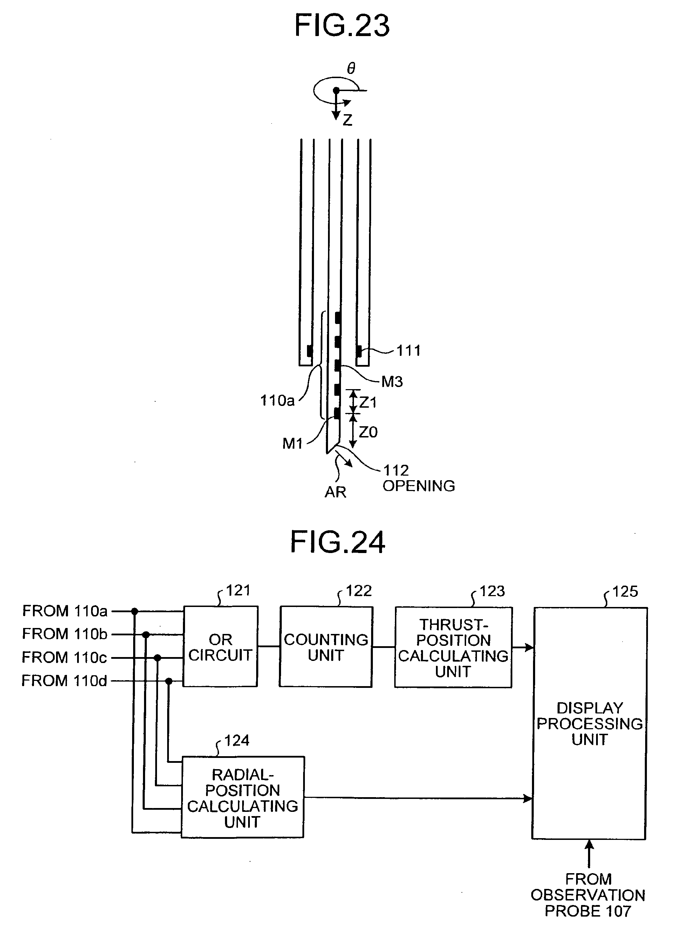

An explanation will be given of the schematic configuration of the end cannula 104, the puncture needle 110, and the observation probe 7 with reference to FIG. 21. FIG. 22 is a cross-sectional view taken along line A-A illustrated in FIG. 21, and FIG. 23 is a cross-sectional view that illustrates the state where the puncture needle 110 is inserted into the end cannula 104. In FIGS. 21 to 23, on the inner wall of the end cannula 104 near the end, there are provided magnetic encoders 111 (111a to 111d) that are distributed at four positions in a circumferential direction and arranged at intervals of 90 degrees in the circumferential direction. Each detection result from the encoders 111a to 111d is output to the external observation-device main body 8.

Both the puncture needle 110 and the observation probe 107 have needle-shaped ends, and a plurality of magnetic markers 110a and 107a, which covers about 90 degrees in a circumferential direction, is arranged at a predetermined pitch Z1 in an axial direction on the outer surfaces near the ends. Therefore, when the puncture needle 110 or the observation probe 107 is inserted into the end cannula 104, as illustrated in FIG. 22, any one or more of the encoders 111a to 111d detects the marker 110a, and, because the marker 110a is detected, it is possible to detect the relative circumferential-direction (radial-direction) position of the puncture needle 110 with respect to the end cannula 104. For example, in FIG. 22, it can be detected that the puncture needle 110 is positioned with respect to the end cannula 104 with the marker 110a at about 45 degrees. Moreover, as illustrated in FIG. 23, the encoders 111a to 111d sequentially detects the markers from the marker M1 on the end side of the puncture needle 110. The position of the marker M1 in an axial direction from an opening 12 at the end, from which the medicinal solution is administered, is known, and the pitch Z1 between the markers 110a is known; therefore, it is possible to detect the relative axial-direction (thrust-direction) position of the end of the puncture needle 110 from the position of the encoder 111. For example, in FIG. 23, it is possible to detect that the end of the puncture needle 110 is positioned at a distance (Z0+Z1×2) from the encoder 111. The relative radial-direction position and thrust-direction position of the observation probe 107 can be detected in the same manner as the puncture needle 110.

As illustrated in FIG. 24, detection outputs from the encoders 111a to 111d are sent to the observation-device main body 8. In the observation-device main body 8, the relative radial-direction position and thrust-direction position of the puncture needle 110 or the observation probe 107 are detected, whereby a three-dimensional cross-sectional image observed by the observation probe 107 and the end position of the puncture needle 110 can be displayed in a superimposed manner.

In FIG. 24, information detected by each of the encoders 110a to 110d is, if at least one of the encoders 110a to 110d is detected by an OR circuit 121, counted by a counting unit 122, and, in accordance with the counting result, a thrust-position calculating unit 123 calculates the end position (the position of the opening 112) of the puncture needle 110 or the end position (the position of a transmitting and receiving unit 107b) of the observation probe 107 with respect to the end cannula 104 and outputs it to a display processing unit 125. A radial-position calculating unit 124 calculates the radial-direction position in which the marker 110a or 107a is arranged in accordance with the detection result of each of the encoders 110a to 110d and outputs the result to the display processing unit 125. Observation images are input to the display processing unit 125 from the observation probe 107 in advance or afterwards. The display processing unit 125 displays and outputs an observation image on the relative three-dimensional coordinates with respect to the end cannula 104 on the basis of the relative thrust direction and the relative radial direction of the observation probe 107 with respect to the end cannula 104. Moreover, if the relative thrust direction and the relative radial direction of the puncture needle 110 with respect to the end cannula 104 are obtained, the end thrust position of the puncture needle 110 and the radial direction of the opening 112 are displayed in a superimposed manner on the observation image.

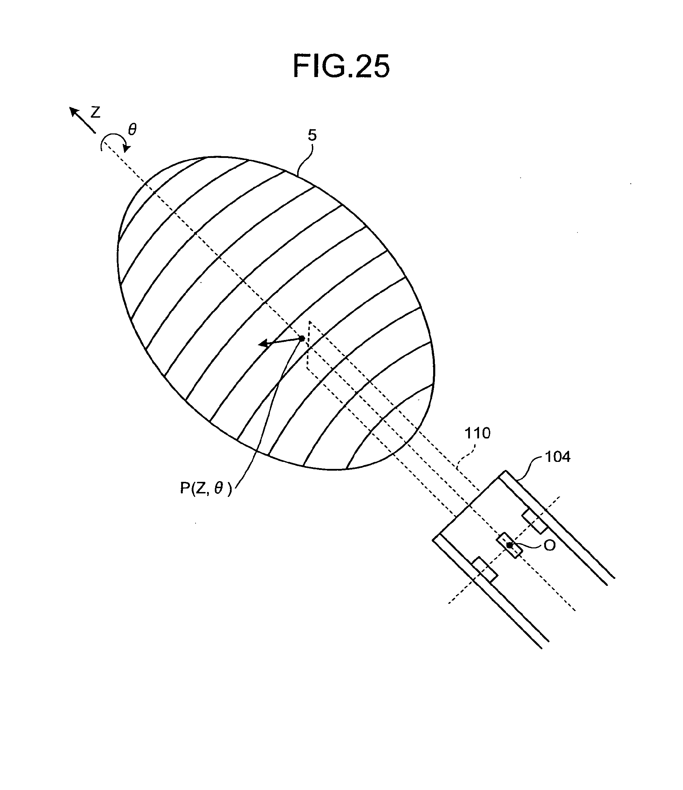

For example, like the display screen illustrated in FIG. 25, a processing target 5 is three-dimensionally displayed by the ultrasonic cross-sectional image on the display screen, and the thrust-direction position Z and the radial-direction position θ of the end of the puncture needle 110 is displayed and output in a superimposed manner on the three-dimensionally displayed image. Thus, the position and the direction of the medicinal-solution administration by the puncture needle 110 can be detected reliably and easily, and the effective medicinal-solution administration can be performed on the processing target 5.

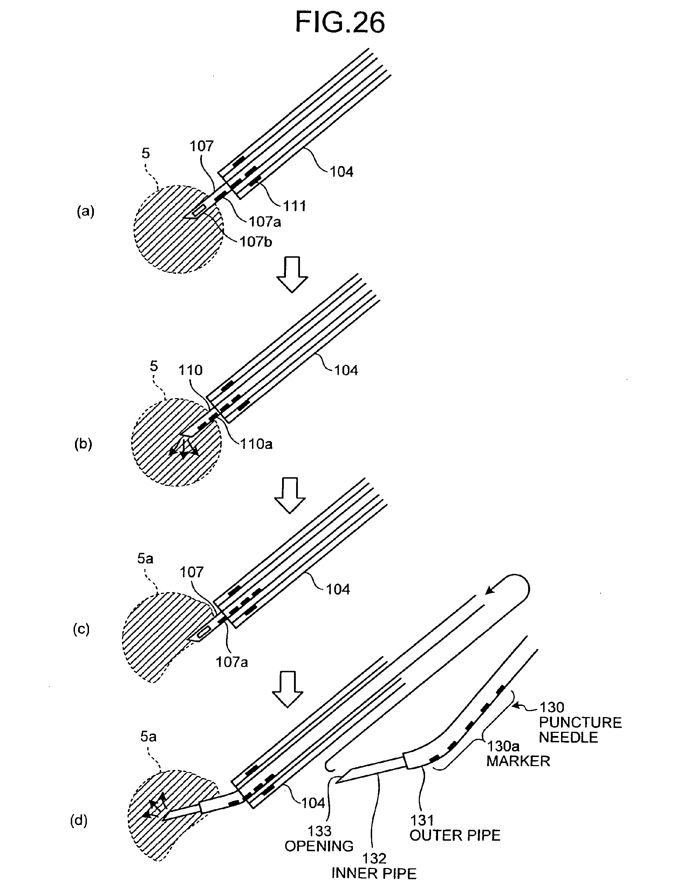

An example of the medicinal-solution administration and the observation by using the puncture needle 110 and the observation probe 107 is illustrated. In FIG. 26, first, the end of the end cannula 104 is tapped into the proximity of the processing target 5, and the observation probe 107 is inserted into the end cannula 104 (FIG. 26(a)). The observation probe 107 is moved to the side of the processing target 5 in a stepwise manner or at a constant speed by the drive unit 117, and, in accordance with the movement, the transmitting and receiving unit 107b is rotated while ultrasonic cross-sectional images are acquired. The relative thrust-direction position and the relative radial-direction position of the observation probe 107 with respect to the end cannula 104 are obtained by the encoders 111a to 111d in accordance with the movement of the observation probe 107. While the observation probe 107 is moved, the ultrasonic cross-sectional image, the relative thrust-direction position, and the relative radial-direction position upon the shooting of the ultrasonic cross-sectional image are detected, whereby the three-dimensional observation image on the relative coordinates with respect to the end cannula 104 can be obtained.

Afterwards, the puncture needle 110 is inserted into the end cannula 104 and the medicinal solution is administered into the processing target 5 (FIG. 26(b)). As described above, the relative thrust-direction position and the relative radial-direction position of the puncture needle 110 with respect to the end cannula 104 are obtained by the encoders 110a to 110d and are sequentially displayed in a superimposed manner on the three-dimensional observation image so that the position and the direction of the medicinal-solution administration to the processing target 5 by the puncture needle 110 can be adjusted reliably and easily.

Furthermore, if the administration of a certain amount of medicinal solution for a certain period of time has finished, the observation probe 7 is inserted into the end cannula 104 again and the three-dimensional observation image of a processing target 5a after the medicinal-solution administration is acquired (FIG. 26(c)). Afterwards, the puncture needle 110 is further inserted into the end cannula 104 and the medicinal-solution administration is performed on the processing target 5a (FIG. 26(d)). In this case, because the three-dimensional observation image of the processing target 5 and the image of the puncture needle 110 are displayed in a superimposed manner on an undepicted monitor, the shape of the processing target 5a and the position of the puncture needle 110 are observed in the three-dimensional observation image while the administration position and the administration direction are guided, by referring to the position of the puncture needle 110 on the three-dimensional observation image, to the position where the medicinal solution can be administered to the processing target 5a in the most effective manner. Thus, even if the shape of the processing target 5a has changed with the passage of time, it is possible to continue the medicinal-solution administration to the processing target 5a in an effective manner. Moreover, as illustrated in FIG. 26(d), when the observation image of the processing target 5a is obtained, the shape of the processing target 5a is referred to and a puncture needle 130 with its end obliquely positioned is used instead of the linear-movement puncture needle 110 so that the invasive end cannula 104 does not need to be inserted and removed.

A multi-layer pipe described in Patent Document 1 is used as the puncture needle 130. Specifically, the puncture needle 130 is a multi-layer pipe formed with at least two or more pipes, an inner pipe 132 is slidably inserted into an outer pipe 131 in the axial direction of the outer pipe 131 and can be protruded from the end of the outer pipe 131, a curved area is formed on the end of at least one pipe, the inner pipe 132 has a smaller bending rigidity than the outer pipe 131, and the curved area is formed to be curved to fall within the limit of elasticity such that the inner pipe 131 is elastically deformable inside the outer pipe 131. A marker 130a that corresponds to the marker 110a, and the like, is arranged near the end of the puncture needle 130.

As a different possible modified example of the puncture needle 130, it is possible to have a configuration such that a needle is attached to the end of a miniature endoscope as the puncture needle 130 so that the medicinal solution is fed via a channel of the endoscope. In this case, a bent area that can be curved (bent) is arranged near the end of the endoscope so that the direction of the needle can be changed.

Although the above-described observation probe 107 obtains the three-dimensional observation images by using the ultrasonic probe as an imaging probe, the present invention is not limited to this and, for example, a bio-sensor probe may be used instead of the observation probe 107 so that the state distribution of the processing targets 5 and 5a are obtained and the optimal position of the medicinal-solution administration is displayed. For example, a transcription factor such as HIF-1 (Hypoxia-Inducible Factor-1) or HLF (HIF-1α Like Factor) is activated because of a hypoxic state and therefore various types of proteins and enzymes are induced such as erythropoietin (EPO) that is an erythropoietic growth factor, a VEGF (Vascular Endothelial Growth Factor) that is a vascular endothelial growth factor and its receptor, endothelin-1 that acts on blood vessels, a plasminogen activator inhibitor-1, iducible nitric oxide synthase, PDGF-B, transferrin that engages with iron metabolism and its receptor, ceruloplasmin, heme oxygenase-1, tyrosine hydroxylase that catalyzes a rate-limiting step in catecholamine biosynthesis, glucose transporter (GLUT1) that introduces glucose to cells, phosphofructokinase that is an enzyme of glycolysis, aldolase A, and enolase (see non-Patent Document 2). It is known that there is a hypoxic state near the center of a tumor, and proteins and enzymes highly expressed due to the hypoxic state are detected as bio-markers of the hypoxic state, whereby it is possible to perform effective medication in which the proximity of the center of a tumor is perceived with precision.

Furthermore, if the ultrasonic probe is used, it is preferable to concurrently use a micro bubble as a contrast agent (see non-Patent Document 3). If the micro bubble is used as a contrast agent, a blood vessel that has a diameter of, for example, 0.2 mm can also be detected so that is it possible to determine the position of a tumor on the basis of angiogenesis induced by canceration.

Moreover, an optical detection probe may be used as an imaging probe instead of the observation probe 107 to determine the size/shape of the processing target 5. For example, an optical CT probe is used as an optical detection probe to acquire optical coherence tomography images (OCT: Optical Coherence Tomography) so that it is possible to determine the size/shape of a tumor although the limit in depth is about several mm at present (see non-Patent Document 4). Furthermore, for example, anti-CEA (carcino embryonic antigen) monoclonal antibody (see Patent Document 2) to which DHE (dihematoporphyrin ether) is bound is used, and a fluorescent probe for detecting fluorescence generated by excitation may be used as an optical detection probe. Qdot(R) may be used as a luminescent molecule of fluorescence (see non-patent document 5). Qdot(R) may be bound to a cancer-specific binding monoclonal antibody. A molecule to which fluorescence is bound is not limited to an antibody, and protein, peptide, nucleic acid, or the like may be used. A fluorescent molecule includes AMCA, SpectrumAqua, FITC, FAM, Rhodamin 6G, Cy(R)3, TRITC, TAMRA, Lissamin(R) rhodamineB, Texas Red(R), Cy(R)5, Cy(R)5.5, Alexa Fluor(R) 350, Pacific Blue(R), Oregon Green(R) 488, Alexa Fluor(R) 488, Alexa Fluor(R) 532, Alexa Fluor(R) 546, Alexa Fluor(R) 555, Alexa Fluor(R) 568, Alexa Fluor(R) 594, Alexa Fluor(R) 647, Alexa Fluor(R) 660, Alexa Fluor(R) 680, and the like. Moreover, for example, a luciferase gene is introduced to cancer cells by a vector and afterwards luciferin is introduced to perform a reaction of excitation, thereby producing luminescence, and a luminescence probe that receives the luminescence may be used as an optical detection probe.

The observation probe 107 that includes the imaging probe described in the third embodiment is what is called an in-depth probe, which means a detector that measures characteristics of an object, and is different from a molecular probe (a collective term for molecules that can find out/search vital functions or recognize a specific molecule) used in a bio/medical system.

Although the above-described end cannula 104 has a configuration such that the observation probe 107 and the puncture needle 110 are alternately inserted into or removed from the end cannula 104, the present invention is not limited to this and the end cannula 104 may have guide holes for a plurality of channels. In this case, the above-described encoders 110a to 110d are arranged on the respective guide holes.

Although each of the encoder 110 and the markers 107a and 110a described above uses magnetism, the present invention is not limited to this and any detecting means of optical-type, capacitance-type, or the like, may be used. Furthermore, it is not limited to a linear-type encoder, and a means that detects the rotation of a ball or a roller moved in accordance with the insertion, removal, or rotation of the observation probe 107 or the puncture needle 110 may be used.

## Claims

1. A medicinal-solution administration system for feeding a medicinal solution to a mammal including a human, the medicinal-solution administration system comprising:
a medicinal-solution feed device that feeds a medicinal solution;
an observation device that includes an elongated observation probe and observes a diseased part to which the medicinal solution is administered; and
a medicinal-solution administration cannula that has at least a part thereof implanted in a body, wherein
the medicinal-solution administration cannula guides the medicinal solution, which is fed from the medicinal-solution feed device, and the observation probe to the diseased part.

2. The medicinal-solution administration system according to claim 1, wherein the medicinal-solution administration cannula has a common channel that is used as a channel for administering the medicinal solution and as a channel for inserting the observation probe, and an administration of the medicinal solution and an observation by the observation probe are performed at different timings.

3. The medicinal-solution administration system according to claim 1, wherein the medicinal-solution administration cannula separately has a channel for administering the medicinal solution and a channel for inserting the observation probe.

4. The medicinal-solution administration system according to claim 3, wherein the channel for administering the medicinal solution and the channel for inserting the observation probe are concentrically arranged.

5. The medicinal-solution administration system according to claim 3, wherein the channel for administering the medicinal solution and the channel for inserting the observation probe are arranged in parallel.

6. The medicinal-solution administration system according to claim 1, wherein the medicinal-solution administration cannula has a side opening and the medicinal solution is administered to the diseased part through the side opening.

7. The medicinal-solution administration system according to claim 6, wherein the medicinal-solution administration cannula has a marker that is to be detected by the observation probe and is arranged near the side opening.

8. The medicinal-solution administration system according to claim 7, wherein
the observation probe is an ultrasonic probe, and
the marker reflects ultrasound generated by the ultrasonic probe.

9. The medicinal-solution administration system according to claim 8, wherein the marker is an air bubble.

10. The medicinal-solution administration system according to claim 6, wherein the medicinal-solution administration cannula is rotatable around an axis of the medicinal-solution administration cannula.

11. The medicinal-solution administration system according to claim 6, wherein the side opening has a plurality of side openings arranged in a circumferential direction.

12. The medicinal-solution administration system according to claim 6, wherein the observation probe is rotatable around an axis of the observation probe while being accommodated in a channel into which the observation probe is inserted in the medicinal-solution administration cannula.

13. The medicinal-solution administration system according to claim 1, wherein
the observation probe is an ultrasonic probe, and
the medicinal-solution administration system includes
a balloon arranged on an end of the probe, and
an inserting unit that inserts fluid into the balloon.

14. The medicinal-solution administration system according to claim 1, wherein the observation probe is an imaging probe.

15. The medicinal-solution administration system according to claim 14, wherein the imaging probe concurrently uses a contrast agent.

16. The medicinal-solution administration system according to claim 14, wherein the imaging probe is a fluorescent probe.

17. The medicinal-solution administration system according to claim 14, wherein the imaging probe is a luminescence probe.

18. The medicinal-solution administration system according to claim 14, wherein the imaging probe is an ultrasonic probe.

19. The medicinal-solution administration system according to claim 1, wherein the observation probe is a bio-sensor probe.

20. The medicinal-solution administration system according to claim 1, wherein
the observation probe includes a marker, and
the medicinal-solution administration cannula includes a detecting unit that detects the marker and is provided in a channel into which the observation probe is inserted.

21. The medicinal-solution administration system according to claim 20, wherein the detecting unit detects a position of the observation probe in a relative radial direction and a relative thrust direction with respect to an axis of the medicinal-solution administration cannula.

22. The medicinal-solution administration system according to claim 20, wherein the marker is a magnetic marker.

23. The medicinal-solution administration system according to claim 1, further comprising an elongated puncture needle that is inserted into the medicinal-solution administration cannula to administer the medicinal solution, wherein
the puncture needle includes a marker, and
the medicinal-solution administration cannula includes a detecting unit that detects the marker and is provided in a channel into which the puncture needle is inserted.

24. The medicinal-solution administration system according to claim 23, wherein the detecting unit detects a position of the puncture needle in a relative radial direction and a relative thrust direction with respect to an axis of the medicinal-solution administration cannula.

25. The medicinal-solution administration system according to claim 23, wherein the marker is a magnetic marker.

26. The medicinal-solution administration system according to claim 23, wherein the puncture needle has a curved area that can be curved near its end.

27. The medicinal-solution administration system according to claim 23, wherein the puncture needle is a multi-layer pipe formed with at least two or more pipes, an inner pipe is slidably inserted into an outer pipe in an axial direction of the outer pipe, the inner pipe can be protruded from an end of the outer pipe, a curved area is formed on an end of at least one pipe, the inner pipe has a smaller bending rigidity than the outer pipe, and the curved area is formed to be curved to fall within a limit of elasticity such that the inner pipe is elastically deformable inside the outer pipe.

28. The medicinal-solution administration system according to claim 23, wherein the observation device displays and outputs a position of the puncture needle in a relative radial direction and a relative thrust direction with respect to an axis of the medicinal-solution administration cannula, which is detected by the detecting unit.

29. The medicinal-solution administration system according to claim 23, wherein display output of the observation device is such that a position of the medicinal-solution administration cannula and a relative position of the puncture needle with respect to the medicinal-solution administration cannula are displayed in a superimposed manner on an observation result of the diseased part.

30. The medicinal-solution administration system according to claim 1, wherein the medicinal-solution feed device includes
a medicinal-solution reservoir that accommodates at least medicinal solution and feeds the medicinal solution to a medicinal-solution administration side;
a variable flow-rate pump that variably discharges to the medicinal-solution reservoir a drive liquid for pushing out the medicinal solution in the medicinal-solution reservoir;
a drive-liquid reservoir that accommodates the drive liquid; and
a control unit that controls a discharge flow rate of the variable flow-rate pump such that a medicinal-solution administration amount per unit of time becomes a predetermined administration amount.

31. A medicinal-solution administration cannula configured to be implanted in a body, configured to guide a medicinal solution fed from a medicinal-solution feed device to a diseased part in order to administer the medicinal solution to the diseased part, and configured to guide an observation probe of the observation device, which observes the diseased part to which the medicinal solution is administered, to proximity of the diseased part in order to observe the diseased part, wherein
the medicinal-solution administration cannula includes a side opening through which the medicinal solution is administered to the diseased part and a marker that is to be detected by the observation probe and is arranged near the side opening.

32. The medicinal-solution administration cannula according to claim 31, wherein
the observation probe is an ultrasonic probe, and
the marker reflects ultrasound generated by the ultrasonic probe.

33. The medicinal-solution administration cannula according to claim 32, wherein the marker is an air bubble.

34. The medicinal-solution administration cannula according to claim 31, wherein the medicinal-solution administration cannula is rotatable around an axis of the medicinal-solution administration cannula.

35. The medicinal-solution administration cannula according to claim 31, wherein the side opening has a plurality of side openings arranged in a circumferential direction.

36. The medicinal-solution administration cannula according to claim 31, wherein a common channel is used as a channel for administering the medicinal solution and as a channel for inserting the observation probe.

37. The medicinal-solution administration cannula according to claim 31, wherein a channel for administering the medicinal solution and a channel for inserting the observation probe are separately provided.

38. The medicinal-solution administration cannula according to claim 31, wherein the channel for administering the medicinal solution and the channel for inserting the observation probe are concentrically arranged.

39. The medicinal-solution administration cannula according to claim 31, wherein the channel for administering the medicinal solution and the channel for inserting the observation probe are arranged in parallel.

40. The medicinal-solution administration cannula according to claim 31, comprising:
an end cannula arranged on a side of the diseased part;
a connection cannula arranged on a side of a medicinal-solution feed port of the medicinal-solution feed device; and
a connection section that connects the end cannula to the connection cannula, wherein
the observation probe is inserted into the end cannula with the connection section disconnected.

41. The medicinal-solution administration cannula according to claim 31, comprising:
an end cannula arranged on a side of the diseased part;
a connection cannula arranged on a side of a medicinal-solution feed port of the medicinal-solution feed device; and
a connection section that connects the end cannula to the connection cannula, wherein
only a channel of the connection section into which the observation probe is inserted is opened so that the observation probe is inserted into the end cannula.

42. The medicinal-solution administration cannula according to claim 40, wherein the end cannula includes
a cylindrical tube that has an end opened, and
an inner-plug member that closes the end of the cylindrical tube.

43. The medicinal-solution administration cannula according to claim 40, wherein the end cannula further includes an outer-casing cannula that is cylindrical and guides the end cannula.
